# EUROPÄISCHE PATENTSCHRIFT

(11) **EP 3 345 989 B1**
(45) Veröffentlichungstag und Bekanntmachung des Hinweises auf die Patenterteilung: **09.09.2026**
(21) Anmeldenummer: 17001889.9
(22) Anmeldetag: 20.11.2017
(51) Int. Cl.: C11D 3/37, C11D 3/39, C11D 7/26, C11D 7/32

(54) **GRANULATE, DEREN VERWENDUNG UND WASCH- UND REINIGUNGSMITTEL ENTHALTEND DIESE**
GRANULES, THEIR USE, AND WASHING AND CLEANING AGENTS CONTAINING THE GRANULES
GRANULÉS, LEUR UTILISATION ET AGENTS DE LAVAGE ET DE NETTOYAGE CONTENANT LESDITS GRANULÉS

(30) Priorität: 31.12.2016 DE 102016015660
(43) Veröffentlichungstag der Anmeldung: 11.07.2018
(73) Patentinhaber: Catexel Production GmbH, 65203 Wiesbaden (DE)
(72) Erfinder: Barreleiro, Paula, 65462 Ginsheim (DE); Morschhäuser, Roman, 55122 Mainz (DE); Kuhse, Bo, 55252 Mainz-Kastel (DE); Ludwig, Rolf, 65817 Eppstein (DE)
(74) Vertreter: Ackermann, Joachim

(56) Entgegenhaltungen:
- WO-A1-2010/115581
- DE-T2- 60 024 463
- DE-T2- 69 104 751
- US-A1- 2008 029 130
- US-A1- 2009 143 272
- US-A1- 2012 151 684
- US-A1- 2013 203 644
- REINHARDT G ET AL: "Oxaziridines and Dioxiranes - Bleach Boosters for Domestic and Industrial Applications", SOFW JOURNAL, VERLAG FUER CHEMISCHE INDUSTRIE H. ZIOLKOWSKY GMBH, DE, vol. 140, no. 9, 1 September 2014 (2014-09-01), pages 48 - 57, XP001591690, ISSN: 0942-7694

## Beschreibung

Die vorliegende Erfindung betrifft Granulate sowie ihre Verwendung in Wasch- und Reinigungsmitteln, insbesondere in Mitteln für die Reinigung von Geschirr.

Um fleckenloses Geschirr zu erhalten, werden in Geschirrspülmitteln für die Reinigung Persalze wie Perborate und Percarbonate eingesetzt. Zur Aktivierung dieser Bleichmittel und um beim Reinigen bei Temperaturen von 60 °C und darunter eine zufriedenstellende Bleichwirkung zu erreichen, enthalten die Geschirrspülmittel in der Regel weiterhin Bleichaktivatoren oder Bleichkatalysatoren, wobei sich insbesondere die Bleichkatalysatoren als besonders wirkungsvoll erwiesen haben.

Bleichkatalysatoren werden in Geschirrspülmitteln für die Reinigung vorzugsweise in Form vorgefertigter Granulate eingesetzt. Dies geschieht zum einen, um ihre Lagerstabilität zu erhöhen, zum anderen aber auch, um eine homogene Einarbeitung winziger Katalysatormengen in die Formulierungen, insbesondere in Tablettenapplikationen, zu gewährleisten.

Sulfonimine und quaternäre Iminiumsalze sind als Bleichkatalysatoren bereits in der Patentliteratur beschrieben worden.

Die DE 691 04 405 T2 entsprechend EP 0 453 003 B1 offenbart Bleichmittel-Zusammensetzungen für Waschmittel, die 1 bis 60 Gew. % einer Persauerstoffverbindung, 0,05 bis 10 Gew. % eines ausgewählten Sulfonimins und 0,5 bis 50 Gew. % eines Tensids enthalten.

In der DE 691 04 751 T2 entsprechend EP 0 446 982 B1 werden Bleichmittel-Zusammensetzungen beschrieben, die 1 bis 60 Gew. % einer Persauerstoffverbindung, 0,05 bis 10 Gew. % eines ausgewählten Sulfonimins und 0,1 bis 40 Gew. % eines mit dem Peroxidanion reagierenden Bleichvorläufers enthalten.

Die DE 696 14 224 T2 entsprechend EP 0 775 192 B1 beschreibt Bleichmittel-Zusammensetzungen, die 1 bis 60 Gew. % einer ausgewählten Persauerstoffverbindung, 0,01 bis 10 Gew. % eines C₁-C₃₀-Imins und 0,001 bis 40 Gew. % eines ausgewählten Übergangsmetallkatalysators enthalten.

Die DE 694 05 407 T2 entsprechend EP 0 728 182 B1 offenbart Bleichmittel-Zusammensetzungen enthaltend 1 bis 60 Gew. % einer Persauerstoffverbindung, 0,01 bis 10 Gew. % eines ausgewählten quaternären Iminiumsalzes als Sauerstoff-Übertragungsmittel und 0,5 bis 50 Gew. % eines Tensids.

Die DE 694 09 252 T2 entsprechend EP 0 728 183 B1 offenbart Bleichmittel-Zusammensetzungen enthaltend 1 bis 60 Gew. % einer Persauerstoffverbindung, 0,01 bis 10 Gew. % eines ausgewählten quaternären Iminiumsalzes als Sauerstoff-Übertragungsmittel und 0,1 bis 40 Gew. % eines Bleichmittel-Vorläufers, der mit einem Peroxid-Anion reagiert.

Bleichkatalysatoren enthaltende Granulate sind bekannt. WO 03/093405 beschreibt Granulate enthaltend Bleichkatalysator, Bleichaktivator und wahlweise eine Beschichtung. Bevorzugte Bleichkatalysatoren zur Herstellung solcher Granulate sind Metallkomplexe mit macrocyclischen, überbrückten Liganden, wie sie in WO 01/64826 und WO 98/39098 beschrieben sind. WO 2010/115581 beschreibt Granulate enthaltend Bleichaktivatoren, Bleichkatalysatoren und mindestens 5 Gew.-% einer oder mehrerer organischen Säuren. Als mögliche Bleichkatalysatoren werden Übergangsmetallsalze bzw. -komplexe des Mangans, Eisens, Kobalts, Rutheniums, Molybdäns, Titans oder Vanadiums genannt.

Im SOFW-Journal 140 9-2014, Seiten 48-57, werden von G. Reinhardt, M. Best, I. Herrgen und M. Ladwig Sulfonimine und quaternäre Iminiumsalze als neuartige Bleichkatalysatoren beschrieben. Diese Verbindungen dienen als Vorläufer von Dioxiranen, Oxaziridinen und Oxaziridiniumsalzen, welche bereits in niedrigen Konzentrationen in Kombination mit Perverbindungen eine ausgezeichnete Bleichwirkung erzielen. Diese Bleichkatalysatoren werden im basischen Millieu eingesetzt, wobei sie ihre größte Wirkung entfalten. Es hat sich herausgestellt, dass Sulfonimine und quaternäre Iminiumsalze bei der Lagerung in fester Form zur Zersetzung neigen. Abgesehen von dem damit einhergehenden Verlust von Wirksubstanz entstehen dabei unerwünschte Verfärbungen. In der oben genannten Veröffentlichung ist bereits vorgeschlagen worden, Sulfonimine und quaternäre Iminiumsalze in der Form von Granulaten mit weiteren Bleichaktivatoren, wie Tetraacetylethylendiamin (TAED) oder Nonanoyloxibenzolsulfonat-Natrium (NOBS), einzusetzen und dabei auch filmbildende Bindemittel zu verwenden. Derartige Granulate sind aber häufig nicht ausreichend lagerstabil, insbesondere bei der Verwendung von Komponenten, welche geringe Mengen an Wasser enthalten.

Überraschenderweise wurde weiterhin gefunden, dass sich Bleichmittel in Kombination mit Sulfoniminen bzw. quaternären Iminiumsalzen zusammen mit weiteren Bleichaktivatoren zur Formulierung von sogenannten Kaltwaschmitteln eignen. Diese Kombinationen gestatten eine hervorragende Bleichwirkung bereits bei niedrigen Temperaturen von z.B. weniger als 35 °C. Außerdem weisen diese Kombinationen neben der Bleichwirkung eine ausgeprägte Tendenz zur Abtötung von Mikroorganismen auf.

Ausgehend von diesem Stand der Technik lag der vorliegenden Erfindung die Aufgabe zugrunde, über längere Zeit lagerstabile Granulate bereitzustellen, die hochaktive Bleichkatalysatoren enthalten, die bei der Lagerung zu keinen Verfärbungen neigen und die keinen nennenswerten Verlust an Aktivsubstanz bei der Lagerung erleiden.

Eine weitere Aufgabe der vorliegenden Erfindung bestand in der Bereitstellung von Granulaten, die in Kombination mit Bleichmitteln hervorragende Waschergebnisse bei niedrigen Temperaturen ermöglichen und die eine drastische Reduktion der Zahl von Mikroorganismen auf den behandelten Substraten bewirken.

Gelöst werden diese Aufgaben durch die Bereitstellung von Granulaten enthaltend
a) 0,1 bis 30 Gew. % eines oder mehrerer Sulfonimine der Formel IV 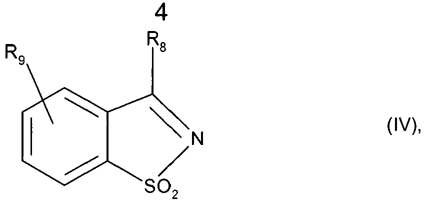 worin R₈ und R₉ unabhängig voneinander Wasserstoff, C₁-C₆-Alkyl oder Phenyl sind.
b) 0,2 bis 5 Gew. % einer oder mehrerer bei 25°C festen, nicht filmbildenden Säuren, die Citronensäure enthalten, und
d) bis zu 80 Gew. % eines oder mehrerer nicht saurer Bindemittel, wobei die Gewichtsangaben auf die Gesamtmenge an Granulat bezogen sind.

Erfindungsgemäß eingesetzte Sulfonimine sind Verbindungen der Formel IV 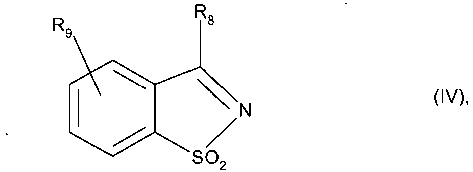 worin R₈ und R₉ unabhängig voneinander Wasserstoff, C₁-C₆-Alkyl oder Phenyl sind. Von den Verbindungen der Formel IV sind solche ganz besonders bevorzugt, worin R₉ Wasserstoff ist und R₈ C₁-C₆-Alkyl, insbesondere Methyl oder Ethyl, oder Phenyl ist.

Äußerst bevorzugtes Sulfonimin der Formel IV ist 3-Methyl-1,2-benzisothiazol-1,1-dioxid.

Bedeutet in dieser Beschreibung einer der Reste Alkyl, so kann die Alkylgruppe sowohl verzweigt als auch unverzweigt sein. Eine Alkylgruppe enthält typischerweise ein bis sechs Kohlenstoffatome. Beispiele für Alkylgruppen sind: Methyl, Ethyl, n-Propyl, Isopropyl, n-Butyl, sec-Butyl, tert.-Butyl, Pentyl, n-Hexyl.

Im erfindungsgemäßen Granulat kommt als Komponente b) Citronensäure zum Einsatz. Im Rahmen der vorliegenden Erfindung umfasst der Begriff "Citronensäure" sowohl die Citronensäure in freier Form als auch in teilneutralisierter Form.

Als Gegenionen von Citronensäure bevorzugt sind Alkaliionen und insbesondere Na-Ionen.

Die Leistung von Bleichmitteln in Wasch- und Reinigungsmitteln kann deutlich vergrößert werden, wenn die Perverbindung mit einer Kombination von Bleichkatalysator mit Bleichaktivator in Kontakt gebracht wird. Hierbei wird die bleichende Wirkung des Katalysators durch die aus dem Aktivator gebildete Peroxycarbonsäure wirksam unterstützt. Zugleich trägt die Peroxycarbonsäure signifikant zur Keimabtötung auf dem zu reinigenden Gut bei, verbessert den Geruch der Waschlauge und unterbindet die Ausbildung eines Biofilms in der Wasch- oder Spülmaschine. Die Kombination von Bleichkatalysatoren und Bleichaktivatoren ist deshalb sinnvoll zur Steigerung der Bleichwirkung und zur Gewährleistung der Hygiene bei der Verwendung von Bleichmitteln in Wasch- und Reinigungsmitteln.

Der Einsatz von Bleichaktivatoren und Bleichkatalysatoren als getrennte Partikel oder Granulate beinhaltet allerdings auch Nachteile, die sich auf die Bleichwirkung negativ auswirken können. Die Reaktionen der Perverbindung bzw. des daraus freigesetzten Wasserstoffperoxyds mit dem Bleichaktivator und dem Bleichkatalysator laufen parallel ab. Löst sich das Bleichkatalysator-Granulat schneller auf als das Bleichaktivator-Granulat, dann ist die Perverbindung bereits verbraucht, bevor diese mit dem Bleichaktivator reagieren kann. Entsprechendes trifft für den umgekehrten Fall zu. Co-Granulate aus Bleichaktivatoren und Bleichkatalysatoren sind weiterhin von Vorteil, um die homogene Verteilung beider Komponenten im Wasch- und Reinigungsmittel zu gewährleisten und um Platz in der Formulierung zu sparen. Weiterhin verbilligen sich die Herstellkosten, da an Stelle zweier verschiedener Granulate nur ein Co-Granulat hergestellt werden muss.

Die Erfindung betrifft daher auch bevorzugt Granulate enthaltend neben den oben beschriebenen Komponenten a), b) und d) einen Bleichaktivator als Komponente c).

Als Bleichaktivatoren können die erfindungsgemäßen Granulate beispielsweise mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyloxi- oder n-Lauroyloxibenzolsulfonat (NOBS bzw. LOBS), acylierte Phenolcarbonsäuren, insbesondere Nonanoyloxi- oder Decanoyloxibenzoesäure (NOBA bzw. DOBA), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran sowie acetyliertes Sorbitol und Mannitol beziehungsweise deren Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam, enthalten. Hydrophil substituierte Acylacetale und Acyllactame werden ebenfalls bevorzugt eingesetzt. Daneben können Nitrilderivate wie n-Methyl-Morpholinium-Acetonitril-Methylsulfat (MMA) oder Cyanomorpholin (MOR) als Bleichaktivatoren Verwendung finden. Auch Kombinationen konventioneller Bleichaktivatoren können eingesetzt werden.

Besonders bevorzugte Bleichaktivatoren sind TAED, NOBS und DOBA.

Als weiteren Inhaltsstoff enthalten die erfindungsgemäßen Granulate ein nicht saures Bindemittel d), um den Zusammenhalt des Granulates zu verbessern.

Die Erfindung betrifft also Granulate enthaltend neben den oben beschriebenen Komponenten a) und b) ein nicht saures Bindemittel als Komponente d).

Die erfindungsgemäßen Granulate können also Kombinationen der Komponenten a), b) und d) oder der Komponenten a), b), c) und d) aufweisen.

Als Bindemittel d) können vorzugsweise Substanzen ausgewählt aus Alkoholalkoxylaten und Polymeren ohne saure Reste verwendet werden. Unter den Polymeren ohne saure Reste werden hierbei synthetische und natürliche Polymere verstanden wie auch modifizierte Polymere natürlichen Ursprungs, welche keine sauren Gruppe, wie Carboxyl- oder Sulfonatgruppen, aufweisen.

Zur Gruppe der mit besonderem Vorzug als Bindemittel d) eingesetzten nichtionischen Polymere zählen Polyvinylalkohole, acetalisierte Polyvinylalkohole, Polyvinylpyrrolidone und Polyalkylenglykole, insbesondere Polyethylenoxide. Bevorzugte Polyvinylalkohole und acetalisierte Polyvinylalkohole weisen Molekulargewichte im Bereich von 10.000 bis 100.000 g/mol, vorzugsweise von 11.000 bis 90.000 g/mol, besonders bevorzugt von 12000 bis 80.000 g/mol und insbesondere bevorzugt von 13.000 bis 70.000 g/mol auf. Bevorzugte Polyethylenoxide haben Molmassen im Bereich von ca. 200 bis 5.000.000 g/mol, entsprechend Polymerisationsgraden n von ca. 5 bis > 100.000.

Weitere als Bindemittel d) bevorzugte Copolymere sind solche, die Struktureinheiten hervorgegangen aus Acrolein und Vinylacetat aufweisen.

In einer weiteren bevorzugten Ausführungsform betrifft die Erfindung Granulate enthaltend neben den oben beschriebenen Komponenten a), b) und d) weitere Zusatzstoffe als Komponente e).

In dieser Ausführungsform können die erfindungsgemäßen Granulate also Kombinationen der Komponenten a), b), d) und e) oder der Komponenten a), b), c), d) und e) aufweisen.

Als weitere Zusatzstoffe e) kommen beispielsweise Sikkative, wie Kalziumsulfat, in Betracht.

In der einfachsten Ausführungsform der Erfindung ist das erfindungsgemäße Granulat nicht mit einer Umhüllung (sog. Coating oder Schutzschicht) versehen.

In einer bevorzugten Ausführungsform der Erfindung ist das erfindungsgemäße Granulat zusätzlich mit einer Umhüllung f) versehen, wodurch die Lagerstabilität weiter verbessert wird und das Granulat gegebenenfalls eingefärbt werden kann. Der Anteil der Umhüllung an der Gesamtmenge des Granulats kann in weiten Bereichen schwanken, sollte aber 30 Gew. % nicht überschreiten.

Als Umhüllung bevorzugt sind bei 25°C feste organische Verbindungen mit filmbildenden Eigenschaften, z.B. Wachse, Polyvinylalkohole, Methylcellulose, Carboxymethylcellulose, Hydroxymethylpropylcellulose, Hydroxymethylcellulose, Hydroxypropylcellulose oder filmbildende Säuren und/oder als Bindemittel d) verwendeten Substanzen. Optional kann die Umhüllung zusätzlich geringe Mengen an wasserlöslichen oder wasserunlöslichen organischen Farbstoffen enthalten.

In einer weiteren bevorzugten Ausführungsform ist das erfindungsgemäße Granulat mit zwei oder mehr Schutzschichten versehen.

In einer sehr bevorzugten Ausführungsform ist das erfindungsgemäße Granulat mit einer ersten Schutzschicht, vorzugsweise aus Hydroxypropylmethylcellulose (HPMC) und/oder aus Methylcellulose (MC), versehen und mit einer zweiten Schutzschicht, vorzugsweise aus einer Fettsäure oder einem Fettsäuregemisch, ganz besonders bevorzugt aus Stearinsäure und Palmitinsäure.

Die Menge an Komponente a) im erfindungsgemäßen Granulat beträgt üblicherweise 0,1 bis 30 Gew. %, bezogen auf die Gesamtmenge an Granulat. Bevorzugte Mengen an Komponente a) bewegen sich im Bereich von 2 bis 12 Gew. %.

Die Menge an Komponente b) im erfindungsgemäßen Granulat beträgt üblicherweise 0,2 bis 5 Gew. %, bezogen auf die Gesamtmenge an Granulat. Bevorzugte Mengen an bei 25 °C festen Säuren, die filmbildend sind, bewegen sich im Bereich von 5 bis 30 Gew. %.

Die Menge an Komponente c) im erfindungsgemäßen Granulat beträgt üblicherweise 0 bis 80 Gew. %, bezogen auf die Gesamtmenge an Granulat. Bevorzugte Mengen an Komponente c) bewegen sich im Bereich von 40 bis 80 Gew. %, insbesondere von 60 bis 80 Gew. %.

Die Menge an Komponente d) im erfindungsgemäßen Granulat beträgt üblicherweise bis zu 80 Gew. %, bezogen auf die Gesamtmenge an Granulat. Bevorzugte Mengen an Komponente d) bewegen sich im Bereich von 15 bis 75 Gew. %, insbesondere von 20 bis 55 Gew. %.

Die Menge an Komponente e) im erfindungsgemäßen Granulat beträgt üblicherweise 0 bis 25 Gew. %, vorzugsweise 0,1 bis 25 Gew. %, bezogen auf die Gesamtmenge an Granulat. Bevorzugte Mengen an Komponente e) bewegen sich im Bereich von 2 bis 20 Gew. %.

Die Menge an Komponente f) im erfindungsgemäßen Granulat beträgt üblicherweise 0 bis 30 Gew. %, bezogen auf die Gesamtmenge an Granulat. Bevorzugte Mengen an Komponente a) bewegen sich im Bereich von 0,5 bis 10 Gew. %.

In Bezug auf ihre Leistungsfähigkeit und ihre Lagerstabilität besonders bevorzugt sind erfindungsgemäße Granulate anzusehen, die, jeweils bezogen auf deren Gesamtgewicht,
a) 2 bis 12 Gew. % eines oder mehrerer Bleichkatalysatoren der Formel IV,
b) 0,2 bis 5 Gew. % Citronensäure,
d) 20 bis 55 Gew. % eines oder mehrerer nicht-saurer Bindemittel, und
e) 2 bis 20 Gew. % Sikkative, enthalten.

Besonders bevorzugt enthalten die erfindungsgemäßen Granulate, bezogen auf deren Gesamtgewicht,
a) 2 bis 12 Gew. % eines oder mehrerer Bleichkatalysatoren der Formel IV,
b) 0,2 bis 5 Gew. % Citronensäure,
c) 40 bis 80 Gew.-% eines oder mehrerer eines oder mehrerer Bleichaktivatoren ausgewählt aus der Gruppe TAED, NOBS oder DOBA,
d) 15 bis 55 Gew. % einer oder mehrerer nicht-saurer Bindemittel, und
e) 2 bis 20 Gew. % Sikkative.

Die oben genannten bevorzugten Granulate können keine Umhüllung f) aufweisen oder vorzugsweise eine Umhüllung f) aufweisen, deren Menge, bezogen auf die Gesamtmenge des Granulats 0,5 bis 10 Gew. % beträgt.

Zur Herstellung der erfindungsgemäßen Granulate sind grundsätzlich verschiedene Granulierverfahren möglich.

Nach einem bevorzugten Herstellverfahren werden die pulverförmigen Aktivsubstanzen zunächst gemischt und dann wird diese Mischung kompaktiert, danach gemahlen und anschließend gegebenenfalls in einzelne Kornfraktionen gesiebt. Die Kompaktierung wird vorzugsweise auf sog. Walzenkompaktoren (z. B. von Fa. Hosokawa-Bepex, Alexanderwerk, Köppern) durchgeführt. Durch die Wahl des Walzenprofils lassen sich einerseits stückige Pellets oder Briketts und andererseits Pressschülpen erzeugen. Während die stückigen Presslinge üblicherweise nur noch vom Feinanteil abgetrennt werden, müssen die Schülpen in einer Mühle auf die gewünschte Partikelgröße zerkleinert werden. Typischerweise kommen als Mühlentyp vorzugsweise schonende Mahlapparate, wie z. B. Sieb- und Hammermühlen (z. B. von Fa. Hosokawa-Alpine, Hosokawa-Bepex) oder Walzenstühle (z. B. von Fa. Bauermeister, Bühler) zum Einsatz.

Von dem so erzeugten Granulat werden durch Siebung der Feinkornanteil und gegebenenfalls der Grobkornanteil abgetrennt. Vorzugsweise wird der Grobkornanteil erneut der Mühle zugeführt und der Feinkornanteil erneut der Kompaktierung. Zur Klassierung der Granulate können gängige Siebmaschinen wie z. B. Taumelsieb- oder Vibrationssiebe (z. B. von Fa. Allgaier, Sweco, Vibra) zum Einsatz kommen.

Eine weitere bevorzugte Variante der Herstellung der erfindungsgemäßen Granulate betrifft das Vermischen und Granulieren der Bestandteile in einem Intensivmischer. Von dem so erzeugten Granulat können anschließend durch Siebung der Feinkornanteil und gegebenenfalls der Grobkornanteil abgetrennt werden.

Die so erzeugten erfindungsgemäßen Granulate können in einem separaten Schritt mit einer Umhüllung versehen werden, beispielsweise in einem Wirbelschichtmischer.

Kennzeichnend für die erfindungsgemäßen Granulate ist in erster Linie ihre chemische Zusammensetzung. Gleichwohl hat es sich erwiesen, dass die Wirkung dieser Granulate auch über die Beeinflussung physikalischer Parameter, wie beispielsweise der Teilchengröße, des Feinanteils sowie des Gehalts an Bleichkatalysator ausgewählter Siebfraktionen vorteilhaft beeinflusst werden kann.

Bevorzugte erfindungsgemäße Granulate weisen aus diesem Grund eine mittlere Teilchengröße zwischen 0,1 und 1,6 mm, vorzugsweise zwischen 0,2 und 1,2 mm und besonders bevorzugt zwischen 0,3 und 1,0 mm auf, jeweils gemessen durch Siebanalyse.

In besonders bevorzugten erfindungsgemäßen Granulaten bewegt sich die volumenmittlere Größe der Primärteilchen im Bereich von 1 µm bis 150 µm, und die fertigen Granulate weisen eine mittlere Teilchengröße zwischen 0,1 und 1,6 mm auf.

Bevorzugte erfindungsgemäße Granulate sind weiterhin dadurch gekennzeichnet, dass deren Wasseranteil weniger als 5 Gew. % (gemessen nach Karl Fischer), bezogen auf die Gesamtmenge des Granulats, beträgt.

Besonders bevorzugte erfindungsgemäße Granulate weisen einen Wasseranteil von weniger als 3 Gew. %, besonders bevorzugt 0 bis 2 Gew. %, bezogen auf die Gesamtmenge des Granulats.

Die erfindungsgemäßen Granulate eignen sich für den Einsatz in allen Wasch- oder Reinigungsmitteln, wobei sich ihr Einsatz in Mitteln für die Reinigung von Geschirr, insbesondere für die maschinelle Reinigung von Geschirr, als besonders vorteilhaft erwiesen hat.

Es wird angenommen, dass die in den erfindungsgemäßen Granulaten enthaltene Säure dabei eine Schutzfunktion übernimmt und die Reaktion von alkalischen Waschmittelbestandteilen mit den alkaliunbeständigen und Hydrolyse empfindlichen Bleichkatalysatoren und den gegebenenfalls vorliegenden Bleichaktivatoren in den erfindungsgemäßen Granulaten unterbindet.

Weiterer Gegenstand der vorliegenden Erfindung ist daher die Verwendung der erfindungsgemäßen Granulate zur Herstellung von Wasch- und Reinigungsmitteln und vorzugsweise von Mitteln für die Reinigung von Geschirr.

Weiterer Gegenstand der vorliegenden Erfindung sind auch Wasch- und Reinigungsmittel, vorzugsweise Mittel für die Reinigung von Geschirr, enthaltend ein erfindungsgemäßes Granulat.

Erfindungsgemäße Wasch- und Reinigungsmittel enthaltend die Komponenten a), b) und c) lassen sich vorteilhafterweise bei tiefen Temperaturen einsetzen und zeigen bei Temperaturen von weniger als 35 °C, vorzugsweise von weniger als 30 °C und ganz besonders bevorzugt von weniger als 25 °C hervorragende Wasch- und Reinigungsergebnisse. Außerdem zeigen diese Wasch- und Reinigungsmittel eine ausgezeichnete keimabtötende Wirkung. Die Anzahl von Bakterien und Hefen auf den damit behandelten Substraten wird durch diese Mittel deutlich verringert.

Bevorzugte erfindungsgemäße Wasch- und Reinigungsmittel, insbesondere die Mittel für die Reinigung von Geschirr, enthalten die erfindungsgemäßen Granulate in Mengen zwischen 0,1 und 10 Gew.-%, vorzugsweise in Mengen zwischen 0,2 und 8 Gew.-% und besonders bevorzugt in Mengen zwischen 0,5 und 6 Gew.-%.

Die erfindungsgemäßen Wasch- und Reinigungsmittel, insbesondere die Mittel für die Reinigung von Geschirr, die als Granulate, pulver- oder tablettenförmige Feststoffe aber auch in flüssiger oder pastöser Form vorliegen können, können außer dem erfindungsgemäßen Granulat im Prinzip alle bekannten und in derartigen Mitteln üblichen Inhaltsstoffe enthalten. Die erfindungsgemäßen Wasch- und Reinigungsmittel, insbesondere die Mittel für die Reinigung von Geschirr, können insbesondere Buildersubstanzen, Persauerstoffverbindungen, Enzyme, Alkaliträger, oberflächenaktive Tenside, pH-Regulatoren, organische Lösungsmittel und weitere Hilfsstoffe, wie Glaskorrosionsinhibitoren, Silberkorrosionsinhibitoren und Schaumregulatoren enthalten. Die erfindungsgemäßen Granulate sind sowohl zum Einsatz in phosphathaltigen als auch phosphatfreien Formulierungen geeignet.

Besonders bevorzugte Wasch- und Reinigungsmittel, insbesondere Mittel für die Reinigung von Geschirr, enthalten
i) 15 bis 65 Gew.-%, vorzugsweise 20 bis 60 Gew.-% einer wasserlöslichen Builderkomponente,
ii) 5 bis 20 Gew.-%, vorzugsweise 8 bis Gew.-%, einer Persauerstoffverbindung,
iii) 0,5 bis 6 Gew.-% eines erfindungsgemäßen Granulates, und
iv) 0 bis 50 Gew.-% weitere Zusatzstoffe wie Enzyme, Alkaliträger, oberflächenaktive Tenside, pH-Regulatoren, organische Lösungsmittel oder weitere Hilfsstoffe, wie Glaskorrosionsinhibitoren, Silberkorrosionsinhibitoren und Schaumregulatoren, jeweils bezogen auf das Gesamtgewicht des Wasch- und Reinigungsmittels.

Ein derartiges Mittel ist insbesondere niederalkalisch, d. h. seine 1-gewichtsprozentige Lösung weist einen pH-Wert im Bereich von 8 bis 11,5 und vorzugsweise von 9 bis 11 auf.

Als wasserlösliche Builderkomponenten in den erfindungsgemäßen Wasch- und Reinigungsmitteln, insbesondere den Mitteln für die Reinigung von Geschirr, kommen prinzipiell alle in derartigen Mitteln üblicherweise eingesetzten Builder in Frage. Beispiele dafür sind Alkaliphosphate, die in Form ihrer alkalischen, neutralen oder sauren Natrium- oder Kaliumsalze vorliegen können, insbesondere Trinatriumphosphat, Tetranatriumdiphosphat, Dinatriumdihydrogendiphosphat, Pentanatriumtriphosphat, sogenanntes Natriumhexametaphosphat sowie die entsprechenden Kaliumsalze beziehungsweise Gemische aus Natrium- und Kaliumsalzen. Ihre Mengen können im Bereich von 15 bis zu etwa 65 Gew. %, vorzugsweise von 20 bis 60 Gew. %, bezogen auf das gesamte Mittel liegen. Weitere mögliche wasserlösliche Builderkomponenten sind neben Polyphosphonaten und Phosphonatalkylcarboxylaten zum Beispiel organische Polymere nativen oder synthetischen Ursprungs vom Typ der Polycarboxylate, die insbesondere in Hartwasserregionen als Co-Builder wirken. In Betracht kommen beispielsweise Polyacrylsäuren und Copolymere aus Maleinsäureanhydrid und Acrylsäure sowie die Natriumsalze dieser Polymersäuren. Handelsübliche Produkte sind zum Beispiel Sokalan^{®} CP 5, CP 10 und PA 30 der Firma BASF. Zu den als Co-Builder brauchbaren Polymeren nativen Ursprungs gehören beispielsweise oxidierte Stärke und Polyaminosäuren wie Polyglutaminsäure oder Polyasparaginsäure. Weitere mögliche wasserlösliche Builderkomponenten sind natürlich vorkommende Hydroxycarbonsäuren wie zum Beispiel Mono-, Dihydroxybernsteinsäure, alpha-Hydroxypropionsäure und Gluconsäure. Zu den bevorzugten organischen wasserlöslichen Builderkomponenten gehören die Salze der Citronensäure, insbesondere Natriumcitrat. Als Natriumcitrat kommen wasserfreies Trinatriumcitrat und vorzugsweise Trinatriumcitratdihydrat in Betracht. Trinatriumcitratdihydrat kann als fein- oder grobkristallines Pulver eingesetzt werden. In Abhängigkeit vom letztlich in den erfindungsgemäßen Wasch- und Reinigungsmitteln, insbesondere den Mitteln für die Reinigung von Geschirr, eingestellten pH-Wert können auch die zu den genannten Co-Builder-Salzen korrespondierenden Säuren vorliegen. Insbesonders bevorzugte Builderkomponenten in phosphat-freien Formulierungen sind Methylglycindiacetat (MDGA, z. B. Trilon^{®} M, BASF), L-Glutaminsäure, N,N, (biscarboxymethyl)- tertra Natriumsalz(GLDA, Dissolvine^{®} DL, Akzo Nobel), Natrium-Polyaspartate (Baypure^{®}, Lanxess) oder Salze der Iminodibernstaeinsäure (Baypure^{®}, Lanxess).

Bevorzugte in den erfindungsgemäßen Wasch- und Reinigungsmitteln, insbesondere den Mitteln für die Reinigung von Geschirr, eingesetzte Persauerstoffverbindungen sind Perborate und Percarbonate, insbesondere die entsprechenden Natriumsalze dieser Verbindungen.

Zu den in erfindungsgemäßen Wasch- und Reinigungsmitteln, insbesondere den Mitteln für die Reinigung von Geschirr, gegebenenfalls enthaltenen Enzymen gehören Proteasen, Amylasen, Pullulanasen, Cutinasen und/oder Lipasen, beispielsweise Proteasen wie BLAP^{™}, Optimase^{™}, Opticlean^{™}, Maxacal^{™}, Maxapem^{™}, Durazym^{™}, Purafect^{™} OxP, Esperase^{™} und/oder Savinase^{™}, Amylasen wie Termamyl^{™}, Amylase-LT^{™}, Maxamyl^{™}, Duramyl^{™} und/oder Lipasen wie Lipolase^{™}, Lipomax^{™}, Lumafast^{™} und/oder Lipozym^{™}. Die verwendeten Enzyme können an Trägerstoffen adsorbiert und/oder in Hüllsubstanzen eingebettet sein, um sie gegen vorzeitige Inaktivierung zu schützen. Sie sind in den erfindungsgemäßen Wasch- und Reinigungsmitteln, insbesondere den Mitteln für die Reinigung von Geschirr, vorzugsweise in Mengen bis zu 10 Gew.-% und besonders bevorzugt in Mengen von 0,05 bis 5 Gew.-% enthalten, wobei insbesondere bevorzugt gegen oxidativen Abbau stabilisierte Enzyme eingesetzt werden.

Vorzugsweise enthalten die erfindungsgemäßen Wasch- und Reinigungsmittel, insbesondere die Mittel für die Reinigung von Geschirr, die üblichen Alkaliträger wie zum Beispiel Alkalisilikate, Alkalicarbonate und/oder Alkalihydrogencarbonate. Zu den üblicherweise eingesetzten Alkaliträgern zählen Carbonate, Hydrogencarbonate und Alkalisilikate mit einem Molverhältnis SiO₂/M₂O (M = Alkaliatom) von 1 : 1 bis 2,5 : 1. Alkalisilikate können dabei in Mengen von bis zu 40 Gew. %, insbesondere von 3 bis 30 Gew. %, bezogen auf das Gesamtgewicht des Wasch- und Reinigungsmittels, enthalten sein. Das in den erfindungsgemäßen Wasch- und Reinigungsmitteln, insbesondere in den Mitteln für die Reinigung von Geschirr, bevorzugt eingesetzte Alkaliträgersystem ist ein Gemisch aus Carbonat und Hydrogencarbonat, vorzugsweise Natriumcarbonat und -hydrogencarbonat, das in einer Menge von bis zu 50 Gew. % und vorzugsweise von 5 bis 40 Gew. % enthalten sein kann.

In einer weiteren bevorzugten Ausführungsform der Erfindung sind in den erfindungsgemäßen Wasch- und Reinigungsmitteln, insbesondere den Mitteln für die Reinigung von Geschirr, 20 bis 60 Gew. % wasserlöslicher organischer Builder, insbesondere Alkalicitrat, 3 bis 20 Gew. % Alkalicarbonat und 3 bis 40 Gew.-% Alkalidisilikat enthalten.

Den erfindungsgemäßen Wasch- und Reinigungsmitteln, insbesondere den Mitteln für die Reinigung von Geschirr, können gegebenenfalls auch Tenside, insbesondere Aniontenside, zwitterionische Tenside und vorzugsweise schwach schäumende nichtionische Tenside zugesetzt werden, die der besseren Ablösung fetthaltiger Anschmutzungen, als Netzmittel und gegebenenfalls im Rahmen der Herstellung dieser Mittel als Granulierhilfsmittel dienen. Ihre Menge kann bis zu 20 Gew. %, vorzugsweise bis zu 10 Gew. % betragen und liegt besonders bevorzugt im Bereich von 0,5 bis 5 Gew. %, bezogen jeweils auf das Gesamtgewicht des Wasch- und Reinigungsmittels.

Üblicherweise werden insbesondere in Mitteln für die maschinelle Reinigung von Geschirr extrem schaumarme Verbindungen eingesetzt. Hierzu zählen vorzugsweise C₁₂-C₁₈-Alkylpolyethylenglykolpolypropylenglykolether mit jeweils bis zu 8 Mol Ethylenoxid- und Propylenoxideinheiten im Molekül. Man kann aber auch andere bekannt schaumarme nichtionische Tenside verwenden, wie zum Beispiel C₁₂-C₁₈-Alkylpolyethylenglykolpolybutylenglykolether mit jeweils bis zu 8 Mol Ethylenoxid- und Butylenoxideinheiten im Molekül, endgruppenverschlossene Alkylpolyalkylenglykolmischether sowie die zwar schäumenden, aber ökologisch attraktiven C₈-C₁₄- Alkylpolyglucoside mit einem Polymerisierungsgrad von etwa 1 bis 4 und/oder C₁₂-C₁₄-Alkylpolyethylenglykole mit 3 bis 8 Ethylenoxideinheiten im Molekül. Ebenfalls geeignet sind Tenside aus der Familie der Glucamide wie zum Beispiel Alkyl-N-Methyl-Glucamide, in denen der Alkylteil bevorzugt aus einem Fettalkohol mit der C-Kettenlänge C₆-C₁₄ stammt. Es ist teilweise vorteilhaft, wenn die beschriebenen Tenside als Gemische eingesetzt werden, zum Beispiel die Kombination Alkylpolyglykosid mit Fettalkoholethoxylaten oder Glucamid mit Alkylpolyglykosiden. Auch die Anwesenheit von Aminoxiden, Betainen und ethoxlierten Alkylaminen ist möglich.

Zur Einstellung eines gewünschten, sich durch die Mischung der übrigen Komponenten nicht von selbst ergebenden pH-Werts können die erfindungsgemäßen Wasch- und Reinigungsmittel, insbesondere die Mittel für die Reinigung von Geschirr, system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure oder Alkalihydrogensulfate, oder Basen, insbesondere Ammonium- oder Alkalihydroxide, enthalten. Derartige pH-Regulatoren sind in den erfindungsgemäßen Wasch- und Reinigungsmitteln, insbesondere den Mitteln für die Reinigung von Geschirr, vorzugsweise nicht über 10 Gew. % und besonders bevorzugt von 0,5 bis 6 Gew. %, enthalten, bezogen jeweils auf das Gesamtgewicht des Mittels.

Zu den in den erfindungsgemäßen Wasch- und Reinigungsmitteln, insbesondere den Mitteln für die Reinigung von Geschirr, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den erfindungsgemäßen Wasch- und Reinigungsmitteln, insbesondere in den Mitteln für die Reinigung von Geschirr, vorzugsweise in einer Menge nicht über 20 Gew. % und besonders bevorzugt von 1 bis 15 Gew. % vorhanden.

Um Glaskorrosion während des Spülganges zu verhindern, können in den erfindungsgemäßen Wasch- und Reinigungsmitteln, insbesondere den Mitteln für die Reinigung von Geschirr, entsprechende Inhibitoren eingesetzt werden. Besonders vorteilhaft sind hier kristalline schichtförmige Silikate und/oder Zinksalze. Die kristallinen schichtförmigen Silikate werden beispielsweise von der Fa. WeylChem Wiesbaden GmbH unter dem Handelsnamen Na-SKS vertrieben, z. B. Na-SKS-1 (Na₂Si₂₂O₄₅·xH₂O, Kenyait), Na-SKS-2 (Na₂Si₁₄O₂₉·xH₂O, Magadiit), Na-SKS-3 (Na₂Si₈O₁₇·xH₂O) oder Na-SKS-4 (Na₂Si₄O₉·xH₂O, Makatit). Von diesen eignen sich vor allem Na-SKS-5 (alpha-Na₂Si₂O₅), Na-SKS-7 (beta-Na₂Si₂O₅, Natrosilit), Na-SKS-9 (NaHSi₂O₅·H₂O), Na-SKS-10 (NaHSi₂O₅·3H₂O, Kanemit), Na-SKS-11 (t-Na₂Si₂O₅) und Na-SKS-13 (NaHSi₂O₅), insbesondere aber Na-SKS-6 (delta-Na₂Si₂O₅). Einen Überblick über kristalline Schichtsilikate findet sich z. B. in dem in "Seifen-Öle-Fette-Wachse, 116 Jahrgang, Nr. 20/1990", auf den Seiten 805-808 veröffentlichten Artikel.

In einer weiteren bevorzugten Ausführungsform weisen die erfindungsgemäßen Wasch- und Reinigungsmittel, insbesondere die Mittel für die Reinigung von Geschirr, eine Menge des kristallinen schichtförmigen Silikats von 0,1 bis 20 Gew.%, besonders bevorzugt 0,2 bis 15 Gew. % und insbesondere bevorzugt 0,4 bis 10 Gew. %, jeweils bezogen auf das Gesamtgewicht des Mittels, auf.

Zur Unterdrückung der Glaskorrosion können erfindungsgemäße Wasch- und Reinigungsmittel, insbesondere Mittel für die Reinigung von Geschirr, mindestens ein Zink- oder Wismutsalz enthalten, vorzugsweise ausgewählt aus der Gruppe der organischen Zinksalze, besonders bevorzugt ausgewählt aus der Gruppe der löslichen organischen Zinksalze, insbesondere bevorzugt ausgewählt aus der Gruppe der löslichen Zinksalze monomerer oder polymerer organischer Säuren und außerordentlich bevorzugt ausgewählt aus der Gruppe Zinkacetat, Zinkacetylacetonat, Zinkbenzoat, Zinkformiat, Zinklactat, Zinkgluconat, Zinkoxalat, Zinkricinoleat, Zinkabietat, Zinkvalerat und Zink-p-toluolsulfonat. Alternativ oder in Kombination mit diesen Zinksalzen können Wismutsalze wie z. B. Wismutacetate engesetzt werden.

Als bevorzugt gelten im Rahmen der vorliegenden Erfindung dabei erfindungsgemäße Wasch- und Reinigungsmittel, insbesondere Mittel für die Reinigung von Geschirr, bei denen die Menge des Zinksalzes bezogen auf das Gesamtgewicht dieses Mittels 0,1 bis 10 Gew. %, vorzugsweise 0,2 bis 7 Gew. % und besonders bevorzugt 0,4 bis 4 Gew. % beträgt und zwar unabhängig davon welche Zinksalze eingesetzt werden, insbesondere also unabhängig davon, ob organische oder anorganische Zinksalze, lösliche oder nicht lösliche Zinksalze oder deren Mischungen eingesetzt werden.

Um einen Silberkorrosionsschutz zu bewirken, können in den erfindungsgemäßen Wasch- und Reinigungsmitteln, insbesondere in den Mitteln für die Reinigung von Geschirr, Silberkorrosionsinhibitoren eingesetzt werden. Bevorzugte Silberkorrosionsinhibitoren sind organische Sulfide wie Cystin und Cystein, zwei- oder dreiwertige Phenole, gegebenenfalls alkyl- oder arylsubstituierte Triazole wie Benzotriazol, Isocyanursäure, Titan-, Zirkonium-, Hafnium-, Cobalt- oder Cersalze und/oder -komplexe, in denen die genannten Metalle je nach Metall in einer der Oxidationsstufen II, III, IV, V oder VI vorliegen.

Sofern die erfindungsgemäßen Wasch- und Reinigungsmittel, insbesondere die Mittel für die Reinigung von Geschirr, zum Beispiel bei Anwesenheit von Aniontensiden, bei der Anwendung zu stark schäumen, können ihnen noch bis zu 6 Gew. %, vorzugsweise etwa 0,5 bis 4 Gew. % einer schaumunterdrückenden Verbindung, vorzugsweise aus der Gruppe der Silikonöle, Gemische aus Silikonöl und hydrophobierter Kieselsäure, Paraffine, Paraffin-Alkohol-Kombinationen, hydrophobierter Kieselsäure, der Bisfettsäureamide, und sonstiger weiterer bekannter im Handel erhältliche Entschäumer zugesetzt werden.

Die erfindungsgemäßen Wasch- und Reinigungsmittel, insbesondere die Mittel für die Reinigung von Geschirr, können als weitere Inhaltsstoffe beispielsweise aus dem Stand der Technik für derartige Mittel bekannte Sequestrierungsmittel, Elektrolyte, zusätzliche Persauerstoff-Aktivatoren, Farbstoffe oder Duftstoffe, wie z. B. Parfümöle, enthalten.

Die Herstellung der erfindungsgemäßen festen Wasch- und Reinigungsmittel, insbesondere der Mittel für die Reinigung von Geschirr, bietet keine Schwierigkeiten und kann im Prinzip in bekannter Weise, zum Beispiel durch Sprühtrocknung oder Granulation, erfolgen, wobei Persauerstoffverbindung und erfindungsgemäßes Granulat gegebenenfalls später getrennt zugesetzt werden.

Erfindungsgemäße Wasch- und Reinigungsmittel in Form wässriger oder sonstige übliche Lösungsmittel enthaltender Lösungen, insbesondere entsprechende Mittel für die Reinigung von Geschirr, werden besonders vorteilhaft durch einfaches Mischen der Inhaltsstoffe, die in Substanz oder als Lösung in einen automatischen Mischer gegeben werden können, hergestellt.

Die erfindungsgemäßen Wasch- und Reinigungsmittel, insbesondere Mittel für die Reinigung von Geschirr, liegen vorzugsweise als pulverförmige, granulare oder tablettenförmige Präparate vor, die in an sich bekannter Weise, beispielsweise durch Mischen, Granulieren, Walzenkompaktieren und/oder durch Sprühtrocknung von thermisch belastbaren Komponenten und Zumischen der empfindlicheren Komponenten, zu denen insbesondere Enzyme, Bleichmittel und der Bleichkatalysator zu rechnen sind, hergestellt werden können.

Zur Herstellung von erfindungsgemäßen Wasch- und Reinigungsmitteln, insbesondere Mitteln für die Reinigung von Geschirr, in Tablettenform geht man vorzugsweise derart vor, dass man alle Bestandteile in einem Mischer miteinander vermischt und das Gemisch mittels herkömmlicher Tablettenpressen, beispielsweise Exzenterpressen oder Rundläuferpressen, mit Pressdrucken im Bereich von 200 · 10⁵ Pa bis 1500 · 10⁵ Pa verpresst.

Man erhält so problemlos bruchfeste und dennoch unter den bestimmungsgemäßen Einsatzbedingungen ausreichend schnell lösliche Tabletten mit Biegefestigkeiten von normalerweise über 150 N. Vorzugsweise weist eine derart hergestellte Tablette ein Gewicht von 15 bis 40 g, insbesondere von 20 bis 30 g auf, bei einem Durchmesser von 35 bis 40 mm.

Die Herstellung erfindungsgemäßer Wasch- und Reinigungsmittel in Form von nicht staubenden, lagerstabil rieselfähigen Pulvern und/oder Granulaten mit hohen Schüttdichten im Bereich von 800 bis 1.000 g/l, insbesondere entsprechender erfindungsgemäßer Mittel für die Reinigung von Geschirr, kann dadurch erfolgen, dass man in einer ersten Verfahrensteilstufe die Builder-Komponenten mit wenigstens einem Anteil flüssiger Mischungskomponenten unter Erhöhung der Schüttdichte dieses Vorgemisches vermischt und nachfolgend - gewünschtenfalls nach einer Zwischentrocknung - die weiteren Bestandteile des Mittels, darunter das erfindungsgemäße Granulat, mit dem so gewonnenen Vorgemisch vereinigt.

Erfindungsgemäße Mittel für die maschinelle Reinigung von Geschirr können sowohl in Haushaltsgeschirrspülmaschinen wie in gewerblichen Spülmaschinen eingesetzt werden. Die Zugabe erfolgt von Hand oder mittels geeigneter Dosiervorrichtungen. Die Anwendungskonzentrationen in der Reinigungsflotte betragen in der Regel etwa 1 bis 8 g/l, vorzugsweise 2 bis 5 g/l. Entsprechendes gilt für den Einsatz der erfindungsgemäßen Wasch- und Reinigungsmittel.

Ein maschinelles Spülprogramm wird zweckmäßig durch einige auf den Reinigungsgang folgende Zwischenspülgänge mit klarem Wasser und einem Klarspülgang mit einem gebräuchlichen Klarspülmittel ergänzt und beendet. Nach dem Trocknen erhält man beim Einsatz erfindungsgemäßer Mittel ein völlig sauberes und in hygienischer Hinsicht einwandfreies Geschirr.

### Beispiele

In den folgenden Beispielen bedeuten %-Angaben Gewichtsprozent (Gew. %), sofern nicht explizit etwas anderes angegeben ist. Bezüglich der angegebenen relativen Luftfeuchten haben die %-Angaben die übliche Bedeutung.

### Beispiele 1 - 3: Herstellung von Granulaten gemäß der Erfindung

In einem Intensivmischer der Fa. Eirich, Hardheim, wurden die einzelnen Bestandteile der erfindungsgemäßen Zusammensetzungen miteinander vermischt und granuliert. Mengen und Bestandteile der Zusammensetzungen der einzelnen Beispiele sind in der nachfolgenden Tabelle 1 angegeben.

**Tabelle 1: Zusammensetzung der Granulate der Beispiele 1-3**

| Beispiel Nr. | 1 | 2 | 3 |
|---|---|---|---|
| 3-Methyl-1,2-benzisothiazol-1,1-dioxid (%) | 9,8 | 9,1 | 9,1 |
| mikrokristalline Zellulose (%)¹⁾ | 19,8 | 18,4 | 18,4 |
| Maisstärke (%) | | 28,1 | 28,1 |
| Reisstärke (%) | 30,3 | | |
| mikrokristalline Zellulose (%)²⁾ | 10,9 | | 10,1 |
| mikrokristalline Zellulose (%)³⁾ | | 10,1 | |
| Kalziumsulfat (%) | 17,5 | 16,2 | 16,2 |
| Polyvinylalkohol (%)⁴⁾ | 9,1 | 8,5 | 8,5 |
| Zitronensäure (%) | 0,3 | 0,3 | 0,3 |
| Dinatriumhydrogencitrat (%) | 0,3 | 0,3 | 0.3 |

| | | | |
|---|---|---|---|
| ¹⁾ Arbocel^{®} FDY 660 von JRS ²⁾ Vivapur^{®} 200 von JRS ³⁾ Heweten^{®} 101 von JRS ⁴⁾ Poval^{®} 6-88 von Kuraray | | | |

Die auf diese Weise erzeugten Granulate wurden anschließend in einer Wirbelschicht-Beschichtungsvorrichtung der Fa. Glatt mit einem Überzug versehen.

Das Granulat gemäß Beispiel 1 erhielt einen Überzug aus 2,2 Gew.-% Hydroxymethylcellulose.

Das Granulat gemäß Beispiel 2 erhielt einen Überzug aus 9,1 Gew.-% Hydroxymethylcellulose.

Das Granulat gemäß Beispiel 3 erhielt einen Überzug aus 9,1 Gew.-% Hydroxypropylcellulose.

### Anwendungsbeispiele

Zur Überprüfung der chemischen und physikalischen Stabilität der Granulate nach Beispielen 1 bis 3 wurde deren Lagerverhalten in einer typischen Waschpulverformulierung untersucht. Hierzu wurden die Granulate nach Beispielen 1 bis 3 in IEC-A Basiswaschpulver eingearbeitet, so dass die fertige Formulierung 3 % Granulat enthielt. Die Mischung wurde in eine Glasflasche überführt und mit geschlossenem Deckel unter Klimabedingungen (T = 32 °C, 75 % relative Luftfeuchte) über mehrere Tage gelagert. Nach 14 Tagen wurde die Farbe des Granulates beurteilt. Als Vergleich wurde anstelle des Granulats pulverförmiges 3-Methyl-1,2-benzisothiazol-1,1-dioxid in das IEC-A Basiswaschpulver eingearbeitet. Die Ergebnisse sind in der nachfolgenden Tabelle 2 dargestellt.

**Tabelle 2: Ergebnisse der Lagertests**

| **Granulat** | **Farbe** | **Farbe (14 Tage)** |
|---|---|---|
| Beispiel 1 | leicht gelblich | leicht gelblich |
| Beispiel 2 | leicht gelblich | leicht gelblich |
| Beispiel 3 | leicht gelblich | leicht gelblich |
| pulverförmiges 3-Methyl-1,2-benzisothiazol-1,1-dioxid | leicht gelblich | gelb-orange |

## Patentansprüche

1. Granulat enthaltend
a) 0,1 bis 30 Gew. % eines oder mehrerer Sulfonimine der Formel IV 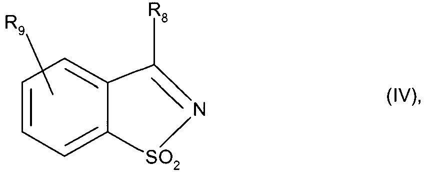 worin R₈ und R₉ unabhängig voneinander Wasserstoff, C₁-C₆-Alkyl oder Phenyl sind.
b) 0,2 bis 5 Gew. % einer oder mehrerer bei 25°C festen, nicht filmbildenden Säuren, die Citronensäure enthalten, und
d) bis zu 80 Gew. % eines oder mehrerer nicht saurer Bindemittel, wobei die Gewichtsangaben auf die Gesamtmenge an Granulat bezogen sind.

2. Granulat nach Anspruch 1, **dadurch gekennzeichnet, dass** R₉ Wasserstoff ist und R₈ C₁-C₆-Alkyl oder Phenyl ist.

3. Granulat nach mindestens einem der Ansprüche 1 bis 2, **dadurch gekennzeichnet, dass** dieses einen oder mehrere Bleichaktivatoren als Komponente c) enthält.

4. Granulat nach mindestens einem der Ansprüche 1 bis 3, **dadurch gekennzeichnet, dass** dieses ein oder mehrere weitere Zusatzstoffe als Komponente e) enthält.

5. Granulat nach mindestens einem der Ansprüche 1 bis 4, **dadurch gekennzeichnet, dass** dieses mit einer Umhüllung als Komponente f) versehen ist.

6. Granulat nach Anspruch 1, **dadurch gekennzeichnet, dass** dieses, jeweils bezogen auf dessen Gesamtgewicht
a) 2 bis 12 Gew. % eines oder mehrerer Bleichkatalysatoren der Formel IV,
b) 0,2 bis 5 Gew. % Citronensäure,
d) 20 bis 55 Gew. % eines oder mehrerer nicht-saurer Bindemittel, und
e) 2 bis 20 Gew. % Sikkative,
enthält.

7. Granulat nach Anspruch 1, **dadurch gekennzeichnet, dass** dieses, bezogen auf dessen Gesamtgewicht,
a) 2 bis 12 Gew. % eines oder mehrerer Bleichkatalysatoren der Formel IV,
b) 0,2 bis 5 Gew. % Citronensäure,
c) 40 bis 80 Gew.-% eines oder mehrerer eines oder mehrerer Bleichaktivatoren ausgewählt aus der Gruppe TAED, NOBS oder DOBA,
d) 15 bis 55 Gew. % einer oder mehrerer nicht-saurer Bindemittel, und
e) 2 bis 20 Gew. % Sikkative
enthält.

8. Granulat nach mindestens einem der Ansprüche 1 bis 7, **dadurch gekennzeichnet, dass** Komponente a) 3-Methyl-1,2-benzisothiazol-1,1-dioxid ist.

9. Verwendung eines Granulats nach mindestens einem der Ansprüche 1 bis 8 zur Herstellung von Wasch- und Reinigungsmitteln.

10. Wasch- und Reinigungsmittel enthaltend ein Granulat nach mindestens einem der Ansprüche 1 bis 8.

11. Wasch- und Reinigungsmittel nach Anspruch 10, **dadurch gekennzeichnet, dass** dieses ein Mittel für die Reinigung von Geschirr ist.

12. Wasch- und Reinigungsmittel nach Anspruch 10, **dadurch gekennzeichnet, dass** dieses neben Sulfoniminen gemäß Anspruch 1als Komponente a) und Citronensäure als Komponente b) weitere Bleichaktivatoren als Komponente c) enthält und sich als Wasch- oder Reinigungsmittel für Temperaturen von weniger als 35 °C eignet.

## Claims

1. Granule comprising
a) 0,1 to 30 weight % of one or more sulfonimines of formula IV 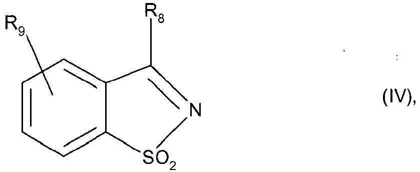 wherein R₈ and R₉ are, independently of one another, hydrogen, C₁-C₆-alkyl, or phenyl,
b) 0.2 to 5% by weight of one or more non-film-forming acids that are solid at 25°C comprising citric acid, and
d) up to 80% by weight of one or more non-acidic binders, wherein the weight figures are based on the total amount of granules.

2. Granule according to claim 1, wherein R₉ is hydrogen and R₈ is C₁-C₆-alkyl or phenyl.

3. Granule according to at least one of claims 1 to 2 containing one or more bleach activators as component c).

4. Granule according to at least one of claims 1 to 3 containing one or more further additives as component e).

5. Granule according to at least one of claims 1 to 4 provided with a coating as component f).

6. Granule according to claim 1 comprising, based in each case on its total weight,
a) 2 to 12 wt.% of one or more bleach catalysts of the formula IV,
b) 0.2 to 5 wt.% citric acid,
d) 20 to 55 wt.% of one or more non-acidic binders, and
e) 2 to 20 wt.% siccatives.

7. Granule according to claim 1 comprising, based on its total weight,
a) 2 to 12 wt.% of one or more bleach catalysts of the formula IV,
b) 0.2 to 5 wt.% citric acid,
c) 40 to 80 wt.% of one or more bleach activators selected from the group consisting of TAED, NOBS, or DOBA,
d) 15 to 55 wt.% of one or more non-acidic binders, and
e) 2 to 20 wt.% siccatives.

8. Granule according to at least one of claims 1 to 7, wherein component a) is 3-methyl-1,2-benzisothiazole-1,1-dioxide.

9. Use of a granule according to at least one of claims 1 to 8 for the production of laundry detergents and cleaning agents.

10. Laundry detergent or cleaning agent comprising a granule according to at least one of claims 1 to 8.

11. Laundry detergent or cleaning agent according to claim 10 which is an agent for cleaning dishware.

12. Laundry detergent or cleaning agent according to claim 10 which, in addition to sulfonimines according to claim 1 as component a) and citric acid as component b), comprises further bleach activators as component c) and is suitable as a laundry detergent or cleaning agent for temperatures of less than 35 °C.

## Revendications

1. Granule comprenant
a) 0,1 à 30 % en poids d'un ou plusieurs sulfonimines de la formule IV 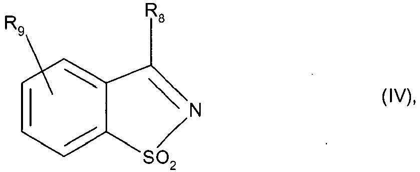 où R₈ et R₉ représentent indépendamment l'hydrogène, un groupe alkyle en C₁-C₆ ou un groupe phényle,
b) 0,2 à 5 % en poids d'un ou plusieurs acides non filmogènes solides à 25 °C contenant de l'acide citrique, et
d) jusqu'à 80 % en poids d'un ou plusieurs liants non acides, les poids étant calculés par rapport à la masse totale des granulés.

2. Granulé selon la revendication 1, **caractérisés en ce que** R₉ représente l'hydrogène et R₈ un groupe alkyle en C₁-C₆ ou un groupe phényle.

3. Granulé selon au moins une des revendications 1 à 2, **caractérisés en ce qu'**ils contiennent un ou plusieurs activateurs de blanchiment comme composant c).

4. Granulé selon au moins une des revendications 1 à 3, **caractérisés en ce qu'**ils contiennent un ou plusieurs additifs supplémentaires comme composant e).

5. Granulé selon au moins une des revendications 1 à 4, **caractérisés en ce qu'**ils sont revêtus comme composant f).

6. Granulé selon la revendication 1, **caractérisés en ce qu'**ils contiennent, par rapport à leur poids total:
a) 2 à 12 % en poids d'un ou plusieurs catalyseurs de blanchiment de formule IV,
b) 0,2 à 5 % en poids d'acide citrique,
d) 20 à 55 % en poids d'un ou plusieurs liants non acides, et
e) 2 à 20 % en poids de siccatifs.

7. Granulé selon la revendication 1, **caractérisés en ce qu'**ils contiennent, par rapport à leur poids total :
a) 2 à 12 % en poids d'un ou plusieurs catalyseurs de blanchiment de formule IV,
b) 0,2 à 5 % en poids d'acide citrique,
c) 40 à 80 % en poids d'un ou plusieurs activateurs de blanchiment choisis parmi le TAED, le NOBS ou le DOBA,
d) 15 à 55 % en poids d'un ou plusieurs liants non acides, et
e) 2 à 20 % en poids de siccatifs.

8. Granulé selon au moins une des revendications 1 à 7, **caractérisés en ce que** le composant a) est le 3-méthyl-1,2-benzisothiazol-1,1-dioxyde,

9. Utilisation de granulé selon au moins une des revendications 1 à 8 pour la production de produits de lavage et de nettoyage.

10. Produit de lavage et de nettoyage comprenant des granulés selon au moins une des revendications 1 à 8.

11. Produit de lavage et de nettoyage selon la revendication 10, **caractérisés en ce qu'**il s'agit d'un produit vaisselle.

12. Produit de lavage et de nettoyage selon la revendication 10, **caractérisés en ce que**, outre les sulfonimines selon la revendication 1 comme composant a) et l'acide citrique comme composant b), ils contiennent également des activateurs de blanchiment comme composant c) et conviennent comme produits de lavage ou de nettoyage à des températures inférieures à 35 °C.
